# EUROPEAN PATENT APPLICATION

(11) **EP 2 486 932 A1**
(43) Date of publication of application: **15.08.2012**
(21) Application number: 11154314.6
(22) Date of filing: 14.02.2011
(51) Int. Cl.: A61K 36/185, A61P 25/00

(54) **Herb extract for cognitive health benefit**

(71) Applicant: Unilever N.V., 3013 AL Rotterdam (NL)
(72) Inventor: The designation of the inventor has not yet been filed
(74) Representative: Acham, Nicholas Clive

(57) **Abstract**

The present invention relates to use of herbal extracts for improved cognitive function, as well as food products having the extracts. A number of studies have, for example, identified positive correlations between childhood IQ, occupation, duration of education in early life, and cognitive function later in life. Various therapeutic agents have been prescribed for impaired cognitive function. We have determined that a combination of an extract of *Terminalia chebula* and *Terminalia bellerica* provides improved and synergistic expression of neuritis, with significantly lower toxicity.

## Description

### FIELD OF INVENTION

The present invention relates to use of herbal extracts for improved cognitive function, as well as food products having the extracts.

### BACKGROUND AND RELATED ART

Any discussion of the prior art throughout the specification should in no way be considered as an admission that such prior art is widely known or forms part of the common general knowledge in the field.

Optimal cognitive health is vital for human beings. Impaired cognitive development is known to have significant effects on quality of life. Cognition is the process of selecting information from the external environment and clearly identifying it, and more specifically; a process of transmitting external stimuli such as visual, auditory, olfactory, gustatory and somatosensory stimuli to the brain through sensory organs, and processing and precisely identifying them by coordinated functioning of multiple regions of the brain. Cognitive abilities consist of two factors; speed of processing information (speed of transforming external stimuli) and the amount of information processing resources (the level of resource allocation for information processing on external stimuli). Therefore, agents that help cognitive development, or which help maintain healthy growth, have wide benefits, especially for children and older people.

Recent years have witnessed an increasing interest in the link between nutrition and cognitive performance in humans, especially, in the impact of nutrition on the development of cognitive abilities during childhood. Similarly, deteriorating cognitive function in late life substantially increases the risk of dementia and other age-related conditions. With the shift to an aging society, the incidence of senile dementia has been increasing and this has become a social problem.

Therefore, these changes are associated with a significant public health burden. A number of studies have, for example, identified positive correlations between childhood IQ, occupation, duration of education in early life, and cognitive function later in life. However these factors are difficult or impossible to manipulate. Consequently there is need for identifying suitable routes for reducing age-related changes in mood and cognitive function such that clinical or nutritional interventions can be minimised.

Neurological networks are made up of individual neurons; each neuron being a separate structural and functional cellular unit. Neurons have special features for the reception of nerve impulses from other neurons, the effect of which may be either excitation or inhibition, and conduction of nerve impulses.

Neurons have long cytoplasmic processes known as neurites, which end in close opposition to the surfaces of other cells. The ends of these neurites are called synaptic terminals and the cell-to-cell contacts they make are known as synapses. The neurites in higher animals are usually specialized to form dendrites and axons which conduct impulses towards and away from the cell body. The arrival of an impulse at a terminal triggers the process of synaptic transmission. This event usually involves the release of a chemical compound from the neuronal cytoplasm invoking a response in the post-synaptic cell. Neurons of the central nervous system consist of discrete segments including the cell body, the dendrites and the axon. While most nerve cells conform to this basic structure, there is a wide range of structural diversity based upon the specific function of the cell within the body.

Neurotrophic factor (NTF) is responsible for survival and function of neurons, and promoting extension of neurites. This factor is an important target for drug development of nerve related diseases.

Various therapeutic agents have been prescribed for impaired cognitive function. Ancient Indian form of medicine (Ayurveda) has recognised the potential of some herbs for various indications, including brain health. Traditional Chinese medications are also available for some of these indications. Of late, these ancient forms of medicines are finding acceptance, not just in the developing and emerging countries, but also in the developed parts of the world, such as Europe and Americas.

*Terminalia chebula (T.chebula)* commonly known as Haritaki is a well known plant. It is used as an ingredient of *Triphala,* an Ayurvedic preparation of a combination of dried fruits of *Terminalia chebula, Terminalia bellerica* and *Emblica officinalis.* Several studies indicate that *Terminalia chebula* may be used as an ingredient in different concoctions. These concoctions have been shown to have antibacterial and antioxidant activities. Further, *Terminalia chebula* may also be used to treat and /or manage gynaecological disorders, diabetes and dental problems. It is also known to improve digestion, promote the absorption of nutrients and regulate the function of the colon.

We have determined that *Terminalia chebula* provides high neurite expression. However, *T.chebula* is quite toxic and cannot be used at higher concentrations, because, in an in-vitro study, we observed that neuronal cells undergo apoptosis when exposed to an extract of fruits of *T.chebula* at concentration higher than 60 ppm. Lower concentrations were found to have limited efficacy.

*Terminalia bellerica* (T bellarica) commonly known as Vibhitaka, which is also a component of Triphala, is known to possess antimalarial and antifungal properties. It is also used as stimulating astringent which may be used in any condition of atony, prolapse, relaxation of the mucosa and for treatment of sore throat and cough. We have found that *Terminalia bellerica* is not as effective as *T.chebula* on neurite growth, but it is less toxic than *T.chebula.*

We have determined that a combination of an extract of *Terminalia chebula* and *Terminalia bellerica* provides improved and synergistic expression of neuritis, with significantly lower toxicity.

### SUMMARY OF THE INVENTION

According to a first aspect, the present invention provides the use of a combination of herbal extracts for improving mental health, said combination comprising a first extract of a part of *Terminalia chebula* and a second extract of a part of *Terminalia bellerica,* wherein ratio of the first extract to the second extract is from 1:1 to 1:2 based on extracted herb material.

According to a second aspect, the present invention provides the use of a combination of herbal extracts of the first aspect for improving mental health by ingesting a daily dose of 50 mg to 2 g of the combination.

According to a third aspect the present invention provides a method of improving mental health of mammals by administering a combination of herbal extracts comprising a first extract of a part of *Terminalia chebula* and a second extract of a part of *Terminalia bellerica,* wherein ratio of the first extract to the second extract is from 1:1 to 1:2 based on extracted herb material.

According to a fourth aspect the present invention provides a food composition for use to improve mental health, comprising a combination of herbal extracts comprising a first extract of a part of *Terminalia chebula* and a second extract of a part of *Terminalia bellerica* wherein ratio of the first extract to the second extract is from 1:1 to 1:2 based on extracted herb material.

According to a fifth aspect the present invention provides a food composition for use to improve mental health, comprising a combination of herbal extracts comprising a first extract of a part of *Terminalia chebula* and a second extract of a part of *Terminalia bellerica* wherein ratio of the first extract to the second extract is from 1:1 to 1:2 based on extracted herb material wherein said composition provides a daily dose of 50 mg to 2 g of the combination.

According to a sixth aspect the present invention provides a medicament for use to improve mental health, comprising a combination of herbal extracts comprising a first extract of a part of *Terminalia chebula* and a second extract of a part of *Terminalia bellerica* wherein ratio of the first extract to the second extract is from 1:1 to 1:2 based on extracted herb material.

According to a seventh aspect the present invention provides the use of a combination of herbal extracts comprising a first extract of a part of *Terminalia chebula* and a second extract of a part of *Terminalia bellerica* in the preparation of a food composition for improving mental health.

According to an eighth aspect the present invention provides a method of improving mental health of mammals by administering a food composition comprising a combination of herbal extracts comprising a first extract of a part of *Terminalia chebula* and a second extract of a part of *Terminalia bellerica;* wherein ratio of the first extract to the second extract is from 1:1 to 1:2 based on extracted herb material.

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above.

Except in the operating and comparative examples, or where otherwise explicitly indicated, all numbers in this description indicating amounts of material ought to be understood as modified by the word "about".

It should be noted that in specifying any range of concentration or amount, any particular upper concentration can be associated with any particular lower concentration or amount.

The terms weight percent, percent by weight, % by weight, wt%, and the like are synonyms that refer to the concentration of a substance as the weight of that substance divided by the weight of the composition and multiplied by 100.

The recitation of numerical ranges by endpoints includes all numbers subsumed within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. Thus, for example, reference to a composition containing "a compound" includes a mixture of two or more compounds. As used in this specification and the appended claims, the term "or" is generally employed in its sense including "and/or" unless the content clearly dictates otherwise.

The term "treatment" or its equivalent, used in this specification, includes prophylactic and therapeutic treatment.

The terms "cognition" and "cognitive function" as used herein are interchangeable and refer to a construct of a range of functions: - Intelligence (fluid, crystallised), - Creativity (generate and open to new ideas, perceive new relationships), - Memory (encoding, storage, retrieval), - Executive functions (dealing with novelty, planning and implementation, monitoring performance, vigilance, inhibition of task irrelevant information), - Cognitive resources (speed of information processing, working memory capacity, attentional capacity).

For a more complete understanding of the above and other features and advantages of the invention, reference should be made to the following detailed description of preferred embodiments.

### DETAILED DESCRIPTION OF THE INVENTION

Improving mental health is a leading indicator of the general well-being of human beings. Improved mental health may be manifested in various forms. This may include improvement of physiology inside the neural cells, improved synaptic connections in the neural cells, improved neural function and improved brain function.

Neural cells are nerve cells such as those that are found in the brain. Such cells are also known as neurons. A neural cell is the basic biochemical cell located within the nervous system. Neural cells come in a variety of different forms; however, the most common delineation between types stems from their function. Sensory neurons are responsible for the brain and nervous system's response mechanisms to stimuli such as light, sound and touch. Motor neurons cause muscle contractions and affect glands when signals are sent from the brain or spinal cord. In addition, inter-neurons are responsible for connecting each neural cell within the various regions of the nervous system. Each neural cell is divided into a variety of different parts. The nucleus is called the soma and is responsible for protein synthesis within the cell. Extensions of the neural cell are called dendrites, the portion responsible for communicating between each neuron with input information. The axon, on the other hand, carries the signals from the nerves away from the soma, connects to the synapse and releases neurotransmitter chemicals to other neurons.

Different regions of the central nervous system are populated by functionally and anatomically distinct synaptic connections. During synapse construction, maintenance and remodelling, it is believed that proteins are selectively transported to these distinct connections. As a result, the specificity of the nervous system is established and modified, at least in part, by these protein targeting mechanisms.

Molecular studies elucidate the biochemistry of plasticity and to link various aspects of synaptic plasticity with behavioural plasticity. One of the important key biochemical entities that participate in the inter and intra-cellular signalling that ultimately produce changes in the structure and function is via activation (that is, phosphorylation) of the transcription factor cAMP response- element- binding protein (CREB). This transcriptional response also depends on NMDA receptor activation. CREB regulates a transcription factor cascade ultimately involved in a growth process that yields synapse-specific structural changes. Neurogenetic studies have shown CREB to be a key control point for LTM (Long term memory) formation. Loss-of-function manipulations of CREB leave learning and STM (short term memory) intact, but impair LTM. It is believed that the combination of herbal extracts leads to stimulation of neuronal cell in a manner similar to NGF (nerve growth factor) where NGF stimulates the genes associated with neurite formation, synaptic connection and function of neurons. The ingredients have been used as CNS (Central nervous system) active agent that stimulates neurite formation.

In a first aspect the invention provides the use of a combination of herbal extracts for improving mental health, said combination comprising a first extract of a part of *Terminalia chebula* and a second extract of a part of *Terminalia bellerica,* wherein ratio of the first extract to the second extract is from 1:1 to 1:2 based on extracted herb material.

*Terminalia chebula* is widely distributed across India. It is abundant from sub-Himalayan terrain to West Bengal, Assam and in all deciduous forests of India, specifically in Madhya Pradesh, Bihar, Assam and Maharashtra. It is found growing at an altitude of 1800 metres in height and 1.5 to 2.5 metre in diameter. Its fruits and other parts are commercial available and the National Biodiversity Act of India has recognised it as a Normally Traded Commodity. *Terminalia chebula* belongs to family Combretaceae.

In traditional Indian Ayurvedic medicine, *Terminalia bellerica* (also from the family Combretaceae) in its fruit form it is used in various preparations. The tree is found in abundance in Madhya Pradesh, Uttar Pradesh, Punjab and Maharashtra in India. Its fruits and other parts are also commercial available and the National Biodiversity Act of India has recognised it too as a Normally Traded Commodity.

The extracts may individually be aqueous, alcoholic or may be hydro-alcoholic. Alternatively, the extracts may be extracts in other solvents such as hexane, chloroform, dilute acids and organic solvents including mixtures thereof. Extraction may be done at any temperature, but preferably in the range of 10 °C to 150 °C, more particularly in the range 55 °C to 95 °C and continued for duration of five minutes to overnight, to obtain desired amount of extract concentrate. It is preferred that the extracts are aqueous, or hydro-alcoholic. Aqueous extracts are preferred as then the extracted herb materials have lower toxicity and the extracts may be easily incorporated in food compositions. Extraction may be done in-house by following well-known methods of solvent extraction. Alternatively, the extracts may also be procured from commercial vendors. While any part of the plants may be used for extraction, it is preferred that the part of *Terminalia chebula* is its fruit. Similarly, it is preferred that the part of *Terminalia bellerica* is its fruit. The term extract includes liquid extracts which contain the soluble herb materials. Extract could also mean dried powder from which traces of solvents have been removed.

Preferably, the invention provides the use of a combination of herbal extracts of the first aspect for improved physiology inside neural cells. This may be due to better survival of cells as observed in cell culture studies. Without wishing to be bound by theory it is believed that *Terminalia chebula* has antioxidant properties, and since oxidative stress is generally injurious to cells, the antioxidants of *Terminalia chebula* help improve physiology inside neural cells. Without wishing to be bound by theory it is believed that the antioxidant property of *Terminalia chebula* is augmented by *Terminalia bellerica.*

Preferably, the invention provides the use of a combination of herbal extracts of the first aspect for improved synaptic connections in neural cells. We believe that neurons show more number of neurites, therefore, they have higher probability of linking to other neurons. Higher synaptic connections may imply better neuronal functions.

Preferably, the invention provides the use of a combination of herbal extracts of the first aspect for improved neural function.

Preferably, the invention provides the use of a combination of herbal extracts of the first aspect for improved brain function.

### Dosage regime

According to another aspect, the present invention provides the use of a combination of herbal extracts as in the first aspect, for improving mental health, by ingesting a daily dose of 50 mg to 2 g of the combination.

The combination of extracts may be ingested in the form of a medicament, such as tablet or capsule, or a food composition or a beverage or a beverage shot, with the daily dosage in the range of 50 mg to 2 g, preferably 100 to 1000 mg per day.

### Method of improving mental health

According to another aspect the present invention provides a method of improving mental health of mammals by administering a combination of herbal extracts comprising a first extract of a part of *Terminalia chebula* and a second extract of a part of *Terminalia bellerica,* wherein ratio of the first extract to the second extract is from 1:1 to 1:2 based on extracted herb material.

### Food composition

According to a further aspect the invention provides a food composition for use to improve mental health, comprising a combination of herbal extracts comprising a first extract of a part of *Terminalia chebula* and a second extract of a part of *Terminalia bellerica* wherein ratio of the first extract to the second extract is from 1:1 to 1:2 based on extracted herb material.

According to yet further aspect the invention provides a food composition for use to improve mental health, comprising a combination of herbal extracts comprising a first extract of a part of *Terminalia chebula* and a second extract of a part of *Terminalia bellerica* wherein ratio of the first extract to the second extract is from 1:1 to 1:2 based on extracted herb material wherein said composition provides a daily dose of 50 mg to 2 g of the combination.

It is preferred that a serving of the food composition provides 5 to 30 % of daily recommended dosage of the combination of herbal extracts.

The food composition may be effective on decline prevention, improvement or enhancement of normal responses of cognitive abilities of a healthy person, or stated differently, they have effects of decline prevention, improvement or enhancement of normal responses of awareness level of a healthy person and have effects of decline prevention, improvement or enhancement of normal responses of discriminatory ability of a healthy person with respect to events (auditory stimuli, visual stimuli, gustatory stimuli, olfactory stimuli, somatosensory stimuli), and are able to exhibit their effects in foods or beverages and medicaments. The term "food" includes beverages.

### Types of food compositions

As used herein, the term "food products" includes both, food products as such, as well as beverages. Suitable food products as such include spreads, dairy products (including milk and yoghurts), desserts, convenience foods/snacks, breakfast cereals and cereal bars, mayonnaises, dressings, sandwich fillings, ready-cook meals, bread, soups, noodles, biscuits, cakes, and frozen confections such as ice creams, water ices and sorbets and yoghurt ice creams. Suitable beverages include tea, tea-flavoured drinks, soy based products, coffee, soft drinks (e.g. carbonated squashes etc) and fruit juice. The food products may be supplemented with higher levels of the extracts of *Terminalia chebula* and *Terminalia bellerica,* so that they may contain higher amounts of the extracts than they would normally contain.

Preferably, the food composition further includes at least one of protein, carbohydrate or fat. It is especially preferred that the food composition includes at least protein and carbohydrate. Alternatively, a source of one of protein, carbohydrate and/or fat can be given as the same time as the food composition, for example, if the food composition does not contain substantial amounts of these ingredients.

Suitable sources of protein include dairy sources such as milk, yoghurt, kefir, cheese, or cream. Other animal sources may also be used depending upon the type of food composition. Alternatively the food composition may include vegetable derived proteins such as soy-protein, rice protein, pea protein or wheat protein.

The amount of protein in the food composition is preferably 0.1 wt% to 30 wt% or 40%, more preferably 0.5 wt% to 25% wt, and most preferably 1 wt% to 20 % wt.

The food compositions may also include one or more carbohydrates, preferably in an amount of from 1 wt% to 90 wt%, more preferably of from 5 wt% to 80% wt, and most preferably 10 wt% to 75% wt. This is preferred to provide energy to the consumer of the food product. Any suitable carbohydrate may be used, for example sucrose, lactose, glucose, fructose, corn syrup, maltodextrins, starch, modified starch or mixtures thereof.

The food composition may also include dietary fibres, for example in an amount of from 0.1 wt% to 10 wt%, more preferably 0.5 wt% to 5 wt%.

The food composition may further include one or more edible fat, preferably up to 40 wt%, more preferably 0.5 wt% to 30 wt%, and most preferably 0.75 wt% to 20 wt%. Any suitable fat may be used with vegetable fats being especially preferred. Suitable examples include vegetable fats, plant oils, nut oils, seed oils, or mixtures thereof. Saturated or unsaturated (mono-unsaturated and poly-unsaturated) fats may be used, although saturated fats are less preferred.

The amounts of protein, fat, carbohydrate and other ingredients in the edible composition will vary according to the product format of the composition and also, where required, according to national or regional legislation.

### Optional ingredients

The food composition may optionally include fruit or vegetable pieces or particles, concentrates, juice or puree, preferably in amounts of up to 60 wt%. Preferably the compositions include 0.1 wt% to 40% wt, more preferably 1 wt% to 20 wt% of these ingredients. The amount of these ingredients will depend upon the type of food composition. The food compositions may also include 0.1 wt% to 15 wt% edible salts, such as sodium chloride, potassium chloride, alkali metal or alkaline earth metal salts of citric acid, lactic acid, benzoic acid, ascorbic acid, or, mixtures thereof. Calcium salts may also be used such as calcium chloride and calcium caseinate. Further optional ingredients include, but not limited to, added vitamins and/or minerals, herbs, spices, flavourings, stabilisers, emulsifiers, aromas, antioxidants, colourants, preservatives or mixtures thereof. These ingredients are used in conventional amounts. These other added vitamins and/or minerals are preferably selected from at least one of vitamins D, E, H, K and calcium, magnesium, potassium, iodine, manganese, molybdenum, phosphorus, selenium and/or chromium.

### Preparation of food composition

The food compositions may be prepared by any suitable method of preparation, including mixing, blending, homogenising, high-pressure homogenising, emulsifying, and dispersing, and/or encapsulating depending upon the type of food composition. It is well within the skill of the person skilled in the art to select the most appropriate preparation method for different types of food compositions.

The food or beverage may be marketed as healthy foods, functional foods, special health care foods, infant foods, or geriatric foods.

### Medicament

In a further aspect the invention provides a medicament for use to improve mental health, comprising a combination of herbal extracts comprising a first extract of a part of *Terminalia chebula* and a second extract of a part of *Terminalia bellerica* wherein ratio of the first extract to the second extract is from 1:1 to 1:2 based on extracted herb material.

The term "medicament" includes pharmaceuticals and quasi drugs designed for the purpose. The medicament is preferably an oral medicament such as tablet, elixir, capsule, pill, lozenge, syrups, suspension or a reconsitutable powder.

According to a further aspect the invention provides the use of a combination of herbal extracts comprising a first extract of a part of *Terminalia chebula* and a second extract of a part of *Terminalia bellerica* in the preparation of a food composition for improving mental health.

### A method of improving mental health

According to yet further aspect the invention provides a method of improving mental health of mammals by administering a food composition comprising a combination of herbal extracts comprising a first extract of a part of *Terminalia chebula* and a second extract of a part of *Terminalia bellerica;* wherein ratio of the first extract to the second extract is from 1:1 to 1:2 based on extracted herb material.

The invention will now be explained with the help of non-limiting examples.

### EXAMPLES

### Example-1: Synergistic combination of Terminalia chebula and Terminalia bellerica

Two grams of pulverised fruit of *Terminalia chebula* were added to 10 ml distilled water. The water was heated to 90 °C, and the temperature was maintained for 1 to 2 hours. The solution was filtered through WHATMAN^{®}filter paper no. 40 and then the filtrate was cooled and an aliquot was tested to determine the total dissolved solids. The solvent was evaporated to get a powder. Five mg was dissolved in 1 ml distilled water to give a 5000 ppm stock solution. This stock solution was diluted further to *lower concentrations for various experiments. Similarly, 5 mg Terminalia bellerica* (powdered *fruit) was* dissolved in distilled 1 ml water to give a 5000 ppm stock solution. This stock solution was diluted further to lower concentrations for various experiments.

### Cell culture

A cell based, high through put screen was designed to assess the effects of herbal extracts on neurite growth. Mouse neuroblastoma Neuro2a (N2a) cells (ATCC no CCL - 131) were grown in medium consisting of Dulbecco's Modified Eagle Medium supplemented with 8% fetal calf serum and antibiotics and were cultured at 37 °C in humidified 95 % air in 5 % carbon-di-oxide incubator.

Prior to treatment, cells were flushed and seeded in 96 well plate with cell density of 2 to 4*104 cells per well and were allowed to attach to the substrate, overnight. Herbal extracts were prepared in media at different concentrations and different combinations. The cells were exposed to the herbal extracts or their combinations for 24 to 28 hours and then cells were observed under an inverted microscope for the changes in neurite growth and morphology of cells.

The numbers of the cells with neurites were counted by a person blind to the experiment following the method of Jagjit S. Gill, et al Molecular Brain Research, vol. 57, pages 123 to 131 (1998*)*. The neurite count in control was normalized and the neurite index for the herbal extracts was calculated and compared to that in control. The results are shown in Table 1.

The toxicity of the herbal extracts was measured using MTT cell viability test at wavelength of 570 nm with the background absorbance at 660 nm using ELISA reader. The cell viability data was normalised to percentage of control and this is also included in Table 1.

Table-1: The effect of the ingredient and their combination on neurite outgrowth and toxicity (cell viability)

**Table-1**

| | ppm | Neurite outgrowth/fold change | % Cell viability |
|---|---|---|---|
| Control | - | 1.00 | 100 |
| *T. chebula* | 20 | 1.49 | 99.4 |
| *T. bellerica* | 20 | 1.39 | 96.5 |
| *T. chebula: T. bellerica* ratio | 1:1 | 2.71 | 102.4 |
| *T.chebula* | 45 | 1.55 | 91.5 |
| *T.bellerica* | 45 | 0.74 | 104.2 |
| *T.chebula: T.bellerica* ratio | 1:1 | 3.30 | 102.2 |
| *T.chebula* | 15 | 1.46 | 99.4 |
| *T. bellerica* | 30 | 1.56 | 94.3 |
| *T.chebula: T.bellerica* ratio | 1:2 | 4.16 | 99.8 |
| *T.chebula* | 20 | 1.46 | 99.4 |
| *T.bellerica* | 40 | 0.74 | 104.2 |
| *T.chebula: T.bellerica* ratio | 1:2 | 4.60 | 106.3 |
| *T.chebula* | 30 | 2.00 | 90.7 |
| *T.bellerica* | 60 | 1.57 | 93.5 |
| *T.chebula: T.bellerica* ratio | 1:2 | 4.34 | 102.8 |
| *T.chebula* | 30 | 2.00 | 90.7 |
| *T.bellerica* | 15 | 1.56 | 96.5 |
| *T.chebula: T.bellerica* ratio | 2:1 | 3.76 | 107.5 |
| *T.chebula* | 40 | 1.55 | 91.5 |
| *T.bellerica* | 20 | 1.39 | 96.5 |
| *T.chebula: T.bellerica* ratio | 2:1 | 3.31 | 102.3 |
| *T.chebula* | 60 | 1.32 | 87.0 |
| *T.bellerica* | 30 | 1.56 | 94.6 |
| *T.chebula: T.bellerica* ratio | 2:1 | 3.11 | 98.3 |

The data in Table-1 indicates that while *Terminalia chebula* provides high neurite growth, it also shows cell toxicity especially at higher ppm levels. This may be seen from the decreasing cell viability as compared to control. Thus *Terminalia chebula* alone does not provide a solution to the problem of providing higher neurite growth with lower cell toxicity. This is solved by a synergistic combination of *Terminalia chebula and Terminalia bellerica* where the combination acts synergistically to increase the neurite outgrowth, while maintaining or improving the cell viability. The benefit of higher neurites is directly proportional to improvement of cognitive health. Higher cell viability is another indicator of cognitive health. Values of cell viability greater than 100 mean that the cells are healthier, and grow better in presence of the combination of herbal extracts, as compared to individual herbs, or where no herb was used.

It will be appreciated that the illustrated examples provides for the use of a combination of herbal extracts for improving mental health, where the combination includes a first extract of a part of *Terminalia chebula* and a second extract of a part of *Terminalia bellerica,* wherein ratio of the first extract to the second extract is from 1:1 to 1:2 based on extracted herb material. It will be appreciated that the illustrated examples provide improved cognitive health, higher neurite expression and show lower neuro-toxicity.

It should be understood that the specific forms of the invention herein illustrated and described are intended to be representative only as certain changes may be made therein without departing from the clear teachings of the disclosure.

Although the invention has been described with reference to specific embodiments, it will be appreciated by those skilled in the art that the invention may be embodied in many other forms.

## Claims

1. The use of a combination of herbal extracts for improving mental health, said combination comprising a first extract of a part of *Terminalia chebula* and a second extract of a part of *Terminalia bellerica,* wherein ratio of the first extract to the second extract is from 1:1 to 1:2 based on extracted herb material.

2. The use as claimed in claim 1 wherein said part of *Terminalia chebula* is its fruit.

3. The use as claimed in claim 1 or 2 wherein said part of *Terminalia bellerica* is its fruit.

4. The use of a combination of herbal extracts as claimed in any one of the preceding claims for improved physiology inside neural cells.

5. The use of a combination of herbal extracts as claimed in any one of the preceding claims for improved synaptic connections in neural cells.

6. The use of a combination of herbal extracts as claimed in any one of the preceding claims for improved neural function.

7. The use of a combination of herbal extracts as claimed in any one of the preceding claims for improved brain function.

8. The use of a combination of herbal extracts as claimed in claim 1 for improving mental health by ingesting a daily dose of 50 mg to 2 g of the combination.

9. A method of improving mental health of mammals by administering a combination of herbal extracts comprising a first extract of a part of *Terminalia chebula* and a second extract of a part of *Terminalia bellerica,* wherein ratio of the first extract to the second extract is from 1:1 to 1:2 based on extracted herb material.

10. A food composition for use as a medicament to improve mental health, said composition comprising a combination of herbal extracts comprising a first extract of a part of *Terminalia chebula* and a second extract of a part of *Terminalia bellerica* wherein ratio of the first extract to the second extract is from 1:1 to 1:2 based on extracted herb material.

11. A food composition for use as a medicament to improve mental health, said composition comprising a combination of herbal extracts comprising a first extract of a part of *Terminalia chebula* and a second extract of a part of *Terminalia bellerica* wherein ratio of the first extract to the second extract is from 1:1 to 1:2 based on extracted herb material, and wherein said composition provides a daily dose of 50 mg to 2 g of the combination.

12. A food composition as claimed in claim 11 wherein a serving of said composition provides 5 to 30 % of daily recommended dosage of said combination of herbal extracts.

13. The use of a combination of herbal extracts comprising a first extract comprising *Terminalia chebula* and a second extract comprising *Terminalia bellerica* in the preparation of a composition for improving mental health.

14. A method of improving mental health of mammals by administering a food composition comprising a combination of herbal extracts comprising a first extract of a part of *Terminalia chebula* and a second extract of a part of *Terminalia bellerica;* wherein ratio of the first extract to the second extract is from 1:1 to 1:2 based on extracted herb material.
